# EUROPEAN PATENT APPLICATION

(11) **EP 1 876 225 A1**
(43) Date of publication of application: **09.01.2008**
(21) Application number: 06116742.5
(22) Date of filing: 06.07.2006
(51) Int. Cl.: C11D 3/00, C11D 3/20, C11D 3/39, C11D 17/04, A01N 25/16, A01N 31/02, A01N 59/00, A61Q 19/02, A61K 8/34, A61K 8/22

(54) **Broad spectrum and skin friendly disinfecting composition**

(71) Applicant: Centennial Ventures BV, 1213 RT Hilversum (NL)
(72) Inventor: Bobbert, Ilja, 3632 AX Loenen aan de Vecht (NL)
(74) Representative: van Heuvel, Margaretha

(57) **Abstract**

The present invention discloses a composition for use in a foam dispenser comprising: 0.1-5% hydrogen peroxide, 21-55% of a C2-C6 alcohol, and 0.01-2% of a foam booster. The foam booster is capable of providing a stable foam when dispensing a composition consisting of 36% ethanol and 0.2% of the foam booster from a foam dispenser. The composition provided as a foam is suitable for disinfecting skin and/or mucus membranes.

## Description

The present invention is directed to ready-to-use skin friendly, broad spectrum disinfectant compositions which include both an alcohol and hydrogen peroxide as active disinfecting constituents.

Infection control is a major concern for health care professionals. Viruses and bacteria on contaminated hands are easily spread among people in health care facilities such as hospitals. Of course, the risk of infection is also present in public places other than hospitals, such as in gyms, washrooms, restaurants, and schools.

A recent trend exists in hospital settings and in food & hospitality sectors towards higher levels of infection control. In addition, an increasing awareness exists for infectious diseases that can be transferred via the skin and respiratory system. These trends have opted the industry to come up with biocidal solutions that can be used more frequently throughout the day. These solutions must be hypoallergenic, non toxic and not produce any residue on the skin that is undesirable.

In certain environments, such as hospitals, the required level of disinfection cannot be achieved by most of the commonly available products. Consequently, specific hand disinfectants have been developed to achieve higher levels of disinfection where the need exists. These types of products generally contain antimicrobial active ingredients like alcohols in high concentrations, iodines/iodophors, chlorhexidine gluconate (CHG), phenolic compounds like parachlorometaxylenol (PCMX) and Triclosan, quaternary ammonium compounds or combinations thereof.

A problem with such products is that they often sacrifice skin mildness for the sake of disinfectant activity or vice versa. For example, while raising the concentration of the active ingredient may lead to a higher level of disinfection, it frequently leads to increased skin irritation.

Parachlorometaxylenol (PCMX) and Triclosan are common phenolic compounds used in antiseptic hand wash solutions. Although PCMX and Triclosan have lower toxicity than other phenols, and are rather mild to the skin, their germicidal activity is low and depends on the formulation ingredients.

Iodine and iodophors have been used in antiseptic hand wash formulations for a long time. Their germicidal activities are low and reduced in the presence of organic matter. Furthermore, these ingredients are toxic and can irritate and stain the skin.

Chlorhexidine gluconate (CHG) is used as a skin cleanser, pre-surgical scrub, germicidal hand rinse and wound cleaner. It is less effective against gram-negative bacteria as compared to gram-positive bacteria and exhibits relatively low germicidal activity.

Alcohols represent a well known group of effective germicides, providing rapid disinfection. They also enhance drying of the disinfecting solution on surfaces or while rubbing the hands, providing for a no-rinse, leave-to-dry application. They also provide for a refreshed feeling on the skin. However, in order to achieve sufficiently high disinfection rates, the disinfecting solution must contain at least 60-70% of the alcohol. These levels of alcohols extremely defat and dry the skin, causing a dry, chapped or cracked skin on repeated use. Damaged skin may even increase the chance of bacterial contamination and presence of bacterial residues, because cracks in the skin are difficult to reach and clean properly. Furthermore, the anti-microbial activity of alcohols tends to drop dramatically when used on wet hands, resulting in insufficient germ kill.

To overcome some of these disadvantages of alcohols, it is known to include additional ingredients in the composition, such as emollients, humectants, and surfactants. For example, US patent 6,617,294 discloses a waterless disinfecting hand cleanser made of a combination of 60 to 90% w/w of an alcohol, silicone based materials, and humectants. These solutions provide for a sticky residue and cannot be used frequently during the day because of the unwanted and unpleasant build up of the silicone materials.

US 5,916,568 discloses a flash-dry disinfectant composition for use as a hand wash comprising 55-80% of a C2-C5 alcohol, 15-35% of a hydrogen peroxide solution providing an effective hydrogen peroxide concentration of about 1.5-3.5% and 5-10% of a bacteriostatic skin emollient. The high alcohol concentration is biocidal and allows quick drying but also causes severe defatting of the skin, especially after repeated use during the day. Also a hydrogen peroxide concentration in the higher ranges, around 2.5-3.5%, is preferred. However, such concentrations bleach and irritate the skin, especially after repeated use during the day. Therefore, this composition is not particularly useful for frequent use on the skin. Furthermore, high alcohol levels are flammable and therefore give rise to safety risks in use and storage.

US 4,900,721 discloses an aqueous disinfectant for disinfecting the skin and mucous membranes comprising 8-25% of a C2-C8 alcohol and 0.2-0.7% hydrogen peroxide. To be sufficiently biocidal, this composition further requires the presence of additional biocidal phenolic compounds and biocidal nitrogen-containing organic compounds, like Chlorhexidine or Vantocil. The latter compounds are undesirable to be used on the skin, especially with more frequent use when build up occurs. Several of the phenolic compounds are also well known as skin allergens, especially when the composition is repeatedly used throughout the day.

US 6,106,774 discloses a ready to use aqueous hard surface cleaning and disinfecting composition comprising 0.1-20% of a C1-6 alcohol and 0.1-10% hydrogen peroxide. The composition further should include 1.0-10% of a glycol ether, or butoxypropanol or propoxypropanol, to provide the desirable stain and soil solubilizing effect. However, the lower ranges of the percentage hydrogen peroxide, i.e. ranges that are suitable for skin application, do not provide a suitably fast disinfection in combination with the higher ranges of the percentage alcohol.

Thus, it is highly desirable to avail of a composition for use on the skin and/or mucous membranes that is able to disinfect within a few minutes, that does not need rinsing after use (thus is suitable for leave-on use), that enables drying by rubbing hands and/or arms, and that is skin friendly in that it does not produce stickiness of the skin after repeated use and is not defatting.

In a first aspect, the present invention provides a biocidal composition for disinfecting skin and/or mucous membranes comprising a C2-C6 alcohol, hydrogen peroxide and a foam booster. It appeared to be highly advantageous to apply the biocidal composition in the form of a foam, in order to provide for exact dosing, to increase contact surface and contact time, and thus to provide a sufficient biocidal efficacy, and to avoid spilling or dripping of the applied dose off the hands, as may be the case in for instance a spray formulation. Thus, the composition of the invention is suitable to produce an instant foam when using a foam dispenser (such as available from Airspray International or Keltec Dispensing Systems).

In particular, the composition comprises 0.1-5% hydrogen peroxide, 21-55% of a C2-C6 alcohol, and 0.01-2% of a foam booster.

Throughout the present invention, percentages are expressed as weight percentages based on total weight of the composition, unless indicated otherwise.

The composition of the invention comprises a C2-C6 alcohol, preferably ethanol, propanol, isopropanol, butanol or 1,3-butanediol, or a mixture thereof, more preferably ethanol, in a concentration (w/w) of 21-55%, preferably of 26-50%, more preferably of 30-45%.

Furthermore the composition comprises hydrogen peroxide as a secondary disinfecting agent. The hydrogen peroxide is provided in an amount of 0.1-5%, preferably 0.2-3%, more preferably 0.2-2%, even more preferably 0.5-2%, most preferably 0.5-1.5% (w/w). A minimum of 0.5% (w/w) hydrogen peroxide typically is required to achieve a hospital strength disinfection level. Higher concentrations than 3% are generally to be avoided when using repeatedly during the day, as it has been observed that such levels may cause skin bleaching.

The composition of the invention further comprises a foam booster for providing a stable foam in the presence of an alcohol, when dispensing the composition from a foam dispenser. In the context of the invention, a foam booster is a highly foaming and/or foam stabilizing surfactant. Foam boosters are commonly known in the art. Preferably, the foam booster used in the composition of the invention provides a stable foam when dispensing a composition consisting of 36% ethanol and 0.2% of the foam booster from a foam dispenser with a relatively high air to liquid ratio on an inert and smooth surface at about 20 °C, preferably a foam dispenser wherein 9-11 ml air is mixed into 1 ml liquid, for instance the L9, L11 or S10 foam dispensers of Airspray International BV. In this way, a relatively "dry" foam is produced.

According to this invention, a stable foam is a foam that remains as a foam for at least 5-10 seconds, preferably for at least 10-15 seconds, more preferably for at least 15-20 seconds, even more preferably for at least 20-25 seconds, most preferably for at least 25-30 seconds, as measured immediately after dispensing from a foam dispenser as mentioned above.

The foam booster is present in a concentration of 0.01-2%, preferably, 0.02-1.5%, more preferably 0.02-1%. The concentration of the foam booster will depend among others on the water and alcohol content of the composition. Generally, an increase in alcohol concentration also requires an increase in foam booster concentration, in order to get a stable foam. However, the foam booster concentration should typically be as low as possible in order to avoid residue build up on the hands, causing stickiness with repeated use. Also, the foam booster should be stable in the presence of alcohol and hydrogen peroxide.

A foam booster may preferably be chosen from at least one of i) nitrogen containing amphoteric or non-ionic surfactants (Amine Oxides, Betaines, Amides and Imidazoline derivatives), ii) C10-C20 alkoxylated fatty alcohols with a degree of alkoxylation of minimal 6, iii) fluoroaliphatic surfactants with a C4-C18 carbon chain containing carbon fluorine (CF2) groups, iv) linear C12-C24 alpha olefin sulfonates and v) C12-C20 sulfosuccinates.

An particularly preferred foam booster is chosen from nitrogen containing amphoteric or non-ionic surfactants and/or C10-C20 alkoxylated fatty alcohols with an alkoxylation degree of minimal 6.

The nitrogen containing amphoteric or non-ionic surfactant comprises at least one amine oxide, betaine and/or amide group and/or is an imidazoline derivative, and further comprises a straight carbon chain containing at least 10 carbon atoms, preferably 10-18 carbon atoms, more preferably 12-18 carbon atoms, attached to the nitrogen. The straight carbon-containing chain may be a fully saturated alkyl or acyl chain, or may be a carbon chain containing a double bond and/or a carbon chain interrupted by a nitrogen and/or oxygen atom, for instance an (additional) amide group. The carbon-containing chain may optionally contain a few (e.g. 1-3) methyl or ethyl substituents. The amine oxides and betaines further contain one or two short chain alkyl groups attached to the nitrogen, preferably ethyl and/or methyl.

These nitrogen containing amphoteric or non-ionic surfactants advantageously may also possess biocidal activity and act synergistically with the hydrogen peroxide and/or alcohol present in the composition.

Preferred amine oxides are C10-C18, preferably C12-C18 alkyl dimethyl amine oxides, such as decyl dimethylamine oxide, lauryl dimethylamine oxide, myristyl dimethylamine oxide, and/or C10-C18, preferably C12-C18 alkyl amidopropyldimethylamine oxides, such as cocamidopropylamine oxide. Preferred betaines are C10-C18 alkyl dimethyl betaines, such as lauryl betaine, and/or C10-C18 alkyl amidopropyl dimethyl betaines, such as capryl/capramidopropyl betaine, cocamidopropyl betaine, oleamidopropyl betaine. Preferred amides are C10-C18 alkyl monoethanol, diethanol and/or triethanol amides, such as cocamide MEA, cocamide DEA and/or cocamide TEA, and/or ethoxylated alkylene amides, such as PEG4 rapeseedamide. Preferred imidazoline derivatives are C10-C18 imidazoline derived amphoteric surfactants, such as sodium cocoamphoacetate, sodium lauroamphoacetate, disodium cocoamphodiacetate, disodium cocoamphodipropionate, and/or disodium lauroamphodiacetate.

The alkoxylated fatty alcohol non-ionic surfactant comprises a fatty alcohol component that is a C10-C20 primary and/or secondary alcohol, preferably a linear C10-C20 primary and/or secondary alcohol, more preferably a linear C10-C20 primary alcohol. The alkoxylated fatty alcohol further has a degree of alkoxylation, preferably ethoxylation, of minimal 6, more preferably has a degree of alkoxylation, preferably ethoxylation, of 6-14. A preferred alkoxylated fatty alcohol non-ionic surfactant is a C12-C18 fatty alcohol ethoxylate with 7 moles of ethylene oxide, such as Cognis Dehydol LT 7.

These alkoxylated fatty alcohol non-ionic surfactants may advantageously also possess a high wetting capacity. The wetting capacity of a surfactant is the capacity to lower the surface tension of water significantly, thereby enhancing the spreading of the aqueous composition over the skin and improving drying of the skin. Because the composition of the invention also contains a significant amount of water, the addition of a foam booster that also has high wetting capacity is advantageous to enhance drying of the skin.

A suitable fluoroaliphatic surfactant with a C4-C18 carbon chain containing carbon fluorine (CF2) groups is C6-16 Perfluoroalkylethyl Phosphate, C8-18 Perfluoroalkylethyl Phosphate, C8-18 Perfluoroalkylethyl Betaine and C4-C18 Perfluoroalkylethyl Thiohydroxypropyltrimonium Chloride, for example the Masurf SF range of Mason Chemical Company.

A suitable linear C12-C24 alpha olefin sulfonates is found in Hansa Group's Hansanil OS (Sodium Olefin C14-C16 Sulfonate).

A suitable sulfosuccinates is a longer chain (C12-C20) amido type sulfosuccinates, such as cocamido MEA or MIPA sulfosuccinate, oleamido MEA or MIPA sulfosuccinate, and the C12-C20 alcohol type sulfosuccinates, such as dioctyl sulfosuccinates and disodium laureth sulfosuccinate.

A single foam booster as well as a mixture of different foam boosters may be used in the composition according to the invention.

The foam booster should further be skin compatible, i.e. not particularly irritating to the skin, eyes or mucosal tissues, and stable in the presence of alcohol.

A few surfactants which are known as good foam boosters are not particularly effective as foam boosters in the compositions of this invention. Foam boosters that do not provide a stable foam according to this invention in the presence of an alcohol are highly foaming surfactants such as dodecyl benzene sulphonic acid and sodium lauryl ether sulphate.

An acid is preferably present in the composition of the invention. The acid may be an inorganic or an organic acid. Mixtures of acids are also contemplated as being useful. The acid is present in an effective amount to establish a targeted pH range for a composition according to the invention. While any number of acids may be used, the acid preferably is a single acid. A particularly preferred acid is a carboxylic acid, such as citric acid, lactic acid, succinic acid, salicylic acid, tartaric acid, sulfamic acid, glutaric acid, 2-furan carboxylic acid, or benzoic acid.

The composition of the invention has a pH of 3-8, preferably a pH of 3-7, more preferably 4-6, most preferably 4-5. Such pH may be maintained, for example, by the inclusion of one or more acids as described herein or a suitable pH buffer.

In order to improve the skin conditioning properties of the composition, the composition further may comprise a skin emollient or moisturizer, such as glycerol, polyglycerol, glycerides, carnithine, sorbitol, lactic acid, castor oil, aloe vera, allantoin, lanolin and its derivatives, and/or cetyl alcohol. Preferably, the skin emollient is used in a concentration of 0.01-2%, more preferably 0.02-1%.

When choosing a foam booster, it may be advantageous to take into account that the foam booster also has skin conditioning properties.

A foam booster with "skin conditioning" properties is able to improve skin moisturization and provide a good after-use feeling of the skin, i.e. has an emollient or softening effect on the skin and prevents dryness of the skin with repeated use. Examples of such foam boosters are non-ionic surfactants with a good wetting capacity which are found within the alkoxylated fatty acid alcohols as described above.

To avoid build up and stickiness of the composition of the invention on the skin, it is preferred that various characteristics of surfactants are combined in as few a number of surfactants as possible, more preferably applied in the lowest feasible concentrations. Therefore, in one embodiment of the invention, foam boosting, wetting and skin conditioning functionalities are included in one surfactant.

Important is that the composition of the invention can be repeatedly applied, in order to comply with the applicable disinfection standards, without any further rinsing, and does not leave substantial amounts of unwanted residues.

Therefore, it is also important that the composition of the invention is as skin compatible as possible. With "skin compatible" is meant that the composition not only has a skin conditioning effect, but also does not produce any form of skin irritation, allergic reaction, and most importantly does not produce any sticky residue on the skin.

The latter is the case with many surfactants which may be generally considered for use on the skin by those skilled in the art. It is important to select a surfactant or surfactant combination which displays the above characteristics. The examples stated in this invention exemplify various compositions that advantageously possess both good skin conditioning properties as well as a good skin compatibility.

Optionally, the composition may comprise 0.05-0.5% of a complexing agent to stabilize the hydrogen peroxide in the composition and to complex or sequester interfering metal ions. Such a hydrogen peroxide stabilizer is preferably a cation sequestering agent and may be chosen from ethylenediaminetetraacetic acid (EDTA), diethylenetriaminepentaacetic acid (DTPA), N-(hydroxyethyl)-ethylenediaminetriacetic acid (HEDTA), nitrilotriacetic acid (NTA), 2-hydroxyethyliminodiacetic acid (HEIDA), and salts thereof or more preferably is chosen from acetanilide, trisodium ethylenediamine disuccinate, phosphonic acid derivatives having 1 to 5 phosphonic acid groups, for instance a Dequest phosphonate (Solutia), 1-hydroxyethylidene-1,1-diphosphonic acid (HEDP), amino tri(methylene phosphonic acid), diethylenetriamine-penta(methylene phosphonic acid), 2-hydroxy ethylimino bis(methylene phosphonic acid), and ethylene diamine tetra(methylene phosphonic acid).

Furthermore, the composition may comprise fragrances, coloring agents and/or solubilizers. In compositions which include a fragrance, it is frequently desirable to include a fragrance solubilizer which assists in the dispersion, dissolution or mixing of the fragrance constituent in an aqueous base. These solubilizers include known compounds, such as condensates of 2 to 30 moles of ethylene oxide with sorbitan mono- and tri-C10-C20 alkanoic acid esters, which are also known as non-ionic surfactants.

Additionally, specific cleaning agents or detergents may optionally be added. Especially when the composition is being applied in a wipe (towellete), the addition of cleaning agents is preferable in order to have the composition clean and disinfect in one. This is particularly useful when the composition is used to clean and disinfect hands.

The balance of the composition consists of deionized water, preferably with a conductivity of less than 5 microSiemens. It will be appreciated that the lower the deionized water conductivity, the longer the shelf life of the product.

In a preferred embodiment, the composition of the invention is essentially free of quaternary ammonium compounds, phenolic compounds, Triclosan/Irgasan, chlorhexidines, and anti-microbial essential oils.

In a second aspect, the invention provides the use of the composition of the first aspect for disinfection and/or sanitization of skin and/or mucous membranes, preferably for disinfection and/or sanitization of hands and arms. The invention provides use of the composition in a foam dispenser, to produce an instant foam that is easily applicable onto skin or mucous membranes.

Use of the composition results in the eradication of, or in reducing the amount of, gram positive or gram negative organisms, fungi, yeasts and enveloped and non-enveloped viruses.

Often the prescription in healthcare settings is to use a certain specific amount of a disinfectant liquid on the skin and remain the surface wetted to allow for a desired contact time. The use of the composition of the invention as a foam advantageously allows a very precise volume to be produced and applied to the skin, a good spreading of the composition on the skin and a precise maintenance of the appropriate contact time, which is important to fulfil disinfection compliance. Furthermore, because the alcohol level is lower than in currently used compositions, the composition of the invention is less likely to evaporate before reaching the required contact time and is less flammable, increasing its practical utility and avoiding usage and storage precautions.

In order to achieve proper disinfection compliance, the product should further allow for repeated usage during the day, at least after each patient or specific activity which may cause infection of the hands. The composition of the invention is advantageously used repeatedly without significant build up of components of the composition on the skin. Practitioners will therefore be stimulated to use the composition of the invention, which also favours disinfection compliance.

### EXAMPLES

Biocidal activity of the exemplified compositions was tested using a controlled bactericidal suspension test conform European Norm for chemical disinfectants and antiseptics EN 1276 (EN 1276: Quantitative suspension test for the evaluation of bactericidal activity of chemical disinfectants and antiseptics used in food, industrial, domestic, and institutional areas: test method and requirements). One ml of a test suspension containing about 10⁸ cfu of the test microorganism per ml is added to 8 ml of the composition to be tested, and 1 ml milli-Q water is added. A clean and dirty condition is simulated by adding bovine albumin serum (0.3% and 3.0% respectively). After 30 seconds or 1 minute contact time, the amount of viable bacteria was determined. The EN 1276 norm prescribes a log 5 reduction in viable count after a contact time of 5 minutes.

### Explanation of Compounds

| | |
|---|---|
| Ethanol Denatured | Commercially available, denatured with a skin compatible and low odor compound |
| Hydrogen Peroxide | Commercially available (Akzo Nobel, Solvay) |
| Dehydol LT 7 | C12-C18 7 moles EO fatty alcohol (Cognis) |
| Tego Betaine F | Cocamidopropyl betaine (Degussa) |
| Tego Betaine F 50 | Cocamidopropyl betaine (Degussa) |
| Rewoteric AMC | Sodium cocoamphoacetate (Degussa) |
| Natrulon H6 | C6 Polyglycerol (Lonza Inc.) |
| Natrulon H10 | C10 polyglycerol(Lonza Inc.) |
| Rewopol SB FA 30 | Disodium Laureth Sulfosuccinate (Degussa) |
| Natrulon RC 50 DG | L-Carnithine and Decaglycerol mixture (Lonza Inc.) |
| Citric Acid | Commercially generally available (various providers) |
| Benzoic Acid | Commercially generally available (various providers) |
| Barlox 10s | N,N-dimethyldecylamine N-oxide (Lonza Inc.) |
| Barlox 12 | Coco alkyldimethyl amine, N-oxide (Lonza Inc.) |
| EDTA | Ethylene Diamine Tetra-acetic Acid |
| Dequest SPE 9505 | Diethylene triamine penta (methylene phosphonic acid) (Solutia) |

### Composition I

38% Ethanol
1.5% Hydrogen Peroxide
0.6% Dehydol LT 7
0.3% Natrulon H-6
0.2% Benzoic Acid
0.1% Barlox 12 (Cocoamine Oxide)
0.1% EDTA
pH ca. 4.5

### Log 10 reduction according to EN 1276 at 1 minute contact time

| | Clean | Dirty |
|---|---|---|
| E. coli ATCC 25922 | > 6.2 | > 6.2 |
| Pseudomonas aeruginosa ATCC 15442 | > 6.2 | > 6.2 |
| Enterococcus hirae ATCC 10541 | > 5.6 | > 5.6 |
| Staphylcoccus aureus ATCC 6538 | > 5.6 | > 5.6 |

### Composition II

35% Ethanol
1% Hydrogen peroxide
0.7% Dehydol LT 7
0.15% Barlox 12
0.2% Benzoic Acid
pH ca. 5

### Log 10 reduction according to EN 1276 at 1 minute contact time

| | Clean | Dirty |
|---|---|---|
| E. coli ATCC 25922 | > 6.2 | > 6.2 |
| Pseudomonas aeruginosa ATCC 15442 | > 6.2 | > 6.2 |
| Enterococcus hirae ATCC 10541 | > 5.6 | > 5.6 |
| Staphylcoccus aureus ATCC 6538 | > 5.6 | > 5.6 |

### Composition III

40% Ethanol
0.7% Hydrogen peroxide
0.6% Dehydol LT 7
0.1% Barlox 10s (Decylamine Oxide)
0.2% Benzoic Acid
0.1% Dequest
pH ca. 4.6

### Log 10 reduction according to EN 1276 at 30 seconds contact time

| | Clean | Dirty |
|---|---|---|
| E. coli ATCC 25922 | 5 | 5 |
| Pseudomonas aeruginosa ATCC 15442 | 5 | 5 |
| Enterococcus hirae ATCC 10541 | 5 | 5 |
| Staphylcoccus aureus ATCC 6538 | 5 | 5 |

Table 1 shows various compositions with a good foam quality and slightly differing skin after-feel.

The reductions in E. coli and Staphylococcus aureus were tested in an EN 1276 suspension test and show the antimicrobial efficacy of the composition on respectively gram negative and gram positive bacteria.

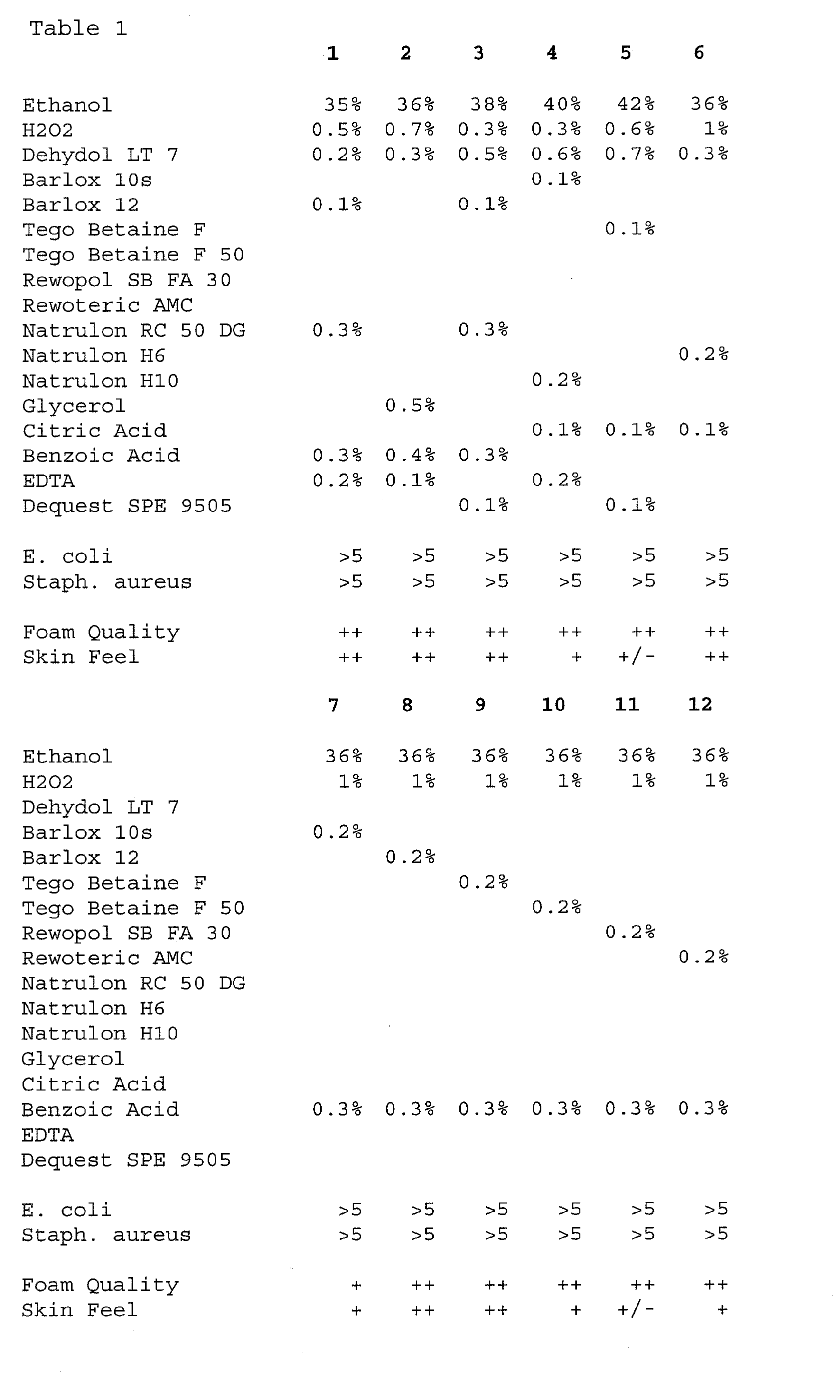

## Claims

1. A composition for providing a foam, the composition comprising:
0.1-5% hydrogen peroxide,
21-55% of a C2-C6 alcohol, and
0.01-2% of a foam booster.

2. The composition of claim 1, wherein the foam is stable for at least 5-10 seconds when dispensing a composition consisting of 36% ethanol and 0.2% of the foam booster from a foam dispenser.

3. The composition of claim 1 or 2, wherein the foam booster is chosen from at least one of i) nitrogen containing amphoteric or non-ionic surfactants, ii) C10-C20 alkoxylated fatty alcohols with a degree of alkoxylation of minimal 6, iii) fluoroaliphatic surfactants with a C4-C18 carbon chain containing carbon fluorine (CF2) groups, iv) linear C12-C24 alpha olefin sulfonates and v) C12-C20 sulfosuccinates.

4. The composition of claim 3, wherein the nitrogen containing amphoteric or non-ionic surfactant is chosen from at least one of i) an amine oxide, preferably a C10-C18 alkyl dimethyl amine oxide and a C10-C18 alkyl amidopropyldimethylamine oxide, ii) a betaine, preferably a C10-C18 alkyl dimethyl betaine and an C10-C18 alkyl amidopropyl dimethyl betaine, iii) an amide, preferably a C10-C18 alkyl monoethanol, diethanol and triethanol amide, and iv) a imidazoline derivative, preferably a C10-C18 imidazoline derivative.

5. The composition of claim 3, wherein the alkoxylated fatty alcohol non-ionic surfactant contains a fatty alcohol component that is a C10-C20 primary and/or secondary alcohol, preferably a linear C10-C20 primary and/or secondary alcohol, and has a degree of alkoxylation of at least 6.

6. The composition of claim 3, wherein the C10-C20 alkoxylated fatty alcohol non-ionic surfactant is an ethoxylated surfactant, preferably having a degree of ethoxylation of at least 6.

7. The composition of any one of claims 1-6 further comprising 0.01-2% of a skin emollient, preferably at least one of glycerol, polyglycerol, glycerides, carnithine, sorbitol, castor oil, aloe vera, allantoin, lanolin and its derivatives, and cetyl alcohol.

8. The composition of any one of claims 1-7 further comprising an inorganic and/or organic acid, such as carboxylic acids or mixtures thereof.

9. The composition of any one of claims 1-8 having a pH in the range of 3-8, preferably a pH of 3-7, more preferably 4-6, most preferably 4-5.

10. Use of the composition of any one of the preceding claims for disinfection and/or sanitization of skin or mucous membranes.

11. Use of the composition of any one of the claims 1-9 in a foam dispenser, in order to produce a foam.

12. Method for disinfection and/or sanitization of the skin or mucous membranes by using a foam comprising the composition of any one of the claims 1-9.

13. Method for producing a foam comprising dispensing the composition of any one of the claims 1-9 from a foam dispenser.
